(19) Europäisches Patentamt

European Patent Office

Office européen des brevets

(11) **EP 0 824 889 A1**

(12) **EUROPEAN PATENT APPLICATION**

(43) Date of publication:
25.02.1998 Bulletin 1998/09

(51) Int. Cl.⁶: **A61B 5/00**

(21) Application number: **97114064.5**

(22) Date of filing: **14.08.1997**

(84) Designated Contracting States:
**AT BE CH DE DK ES FI FR GB GR IE IT LI LU MC NL PT SE**
Designated Extension States:
**AL LT LV RO SI**

(30) Priority: **20.08.1996 IT TO960712**

(71) Applicant:
**Buratto Advanced Technology S.r.l.**
**31035 Crocetta del Montello (Treviso) (IT)**

(72) Inventors:
• **Bedini, Remo**
  **I-56124 Pisa (IT)**

• **Buratto, Alberto**
  **I-31044 Montebelluna ( Treviso) (IT)**
• **Casadio, Gabriele**
  **I-40134 Bologna, (IT)**
• **Palagi, Graziano**
  **Pisa (IT)**
• **Ripoli, Andrea**
  **I-56015 Oratoio, (Pisa) (IT)**

(74) Representative:
**Quinterno, Giuseppe et al**
**c/o JACOBACCI & PERANI S.p.A.**
**Corso Regio Parco, 27**
**10152 Torino (IT)**

(54) **Transmission system using the human body as wave guide**

(57) A communication system (TX, RX) uses the skin (GO) of the human body (CU) as a planar waveguide for the propagation of carried electromagnetic waves. The system comprises a transmitter module (TX) and a receiver module (RX) which can be connected by electrodes (EL) to any two points on the human body (CU) without the use of connections by wires, or magnetic, optical or radio means.

FIG. 1

EP 0 824 889 A1

## Description

The present invention relates in general to apparatus for transmitting data in the form of electrical signals using the human body or part of it as a transmission line. More specifically, the present invention relates to apparatus for transmitting data using a portion of the human body as a waveguide.

The transfer of energy, and hence of electrical signals, in a solid conductor body (to which the human body is comparable) can take place by galvanic conduction and/or by the effect of the electromagnetic field associated with a vector current generated by an excitation system.

An excitation system means a source or a device which can supply electrical signals, for example, an oscillator circuit.

In particular, the effect of variable currents is connected with the so-called "skin effect" which depends mainly on the frequency of the signals concerned.

The transmission, by means of a human body, of an electrical signal applied by means of a galvanic or capacitive connection to the skin of the human body can also take place by the waveguide effect attributable to the structure of the human skin itself.

This principle has been used to set up communication apparatus or a communication system which is the subject of the present invention.

In fact, a need has recently arisen to interconnect several different devices carried on a person's body. These devices may be, for example: watches, electronic processors, portable telephones, pagers, personal stereophonic systems, footwear provided with sensors, computerized footwear, glucometers, cardiac holsters, cardio-frequency meters, etc. An ability to put the various devices carried or worn by a person into communication and hence to integrate their functions has many advantages. For example, the size of the devices can be reduced to the essential minimum by integration of their common functions. For example, a single device provided with a communication interface can be used to control and program all of the devices carried by the person.

Another advantage is that the components of a device can be split up to enable it to be carried more comfortably. For example, an electronic processor or personal computer may have an interface device made in the form of a wristwatch so that it can be consulted extremely conveniently, and may have its central processing unit disposed, for example, in a pocket or attached to the belt for easy carrying.

Another advantage of a communication system of this type is that sensors, for example, biomedical sensors distributed in various parts of the body can be connected to one or more processing and/or display units for processing and/or displaying the data detected by the sensors. For example, footwear may be produced, provided with biomedical sensors communicating with a wrist processing and/or display device.

Naturally, all of these devices could also communicate with one another with the use of conventional systems which are essentially the following: electrical conductors, radio waves, infra-red waves. However, all three of these conventional systems have many disadvantages.

Connection by means of electrical wires is, of course, extremely inconvenient for the person carrying the devices which need to communicate with one another. Moreover, these electrical wires run the risk of being torn or damaged accidentally because of the person's movements.

Infra-red communication systems have the serious disadvantage of permitting communication only between two devices in sight of one another. These conditions can certainly not be guaranteed in the case of devices disposed on any part of the human body.

Devices communicating by radio waves, on the other hand, have the disadvantage of being susceptible to radio interference and disturbances. Since nowadays almost all environments frequented by people are saturated with radio waves and electromagnetic interference, these communication systems would encounter considerable limitations in use. Moreover, these communication systems are themselves sources of radio interference so that their spread would inevitably lead to interference between the communication systems of people who are close together. For this reason, radio communication systems are also unable to ensure communication between the various devices carried by a person. To ensure reliable radio communications it would be necessary to use digital communication systems controlled by complicated protocols which can ensure communication even in the event of disturbances and interference. However, these systems would be very complex and consequently expensive.

The object of the present invention is to provide communication apparatus which solves all of the problems indicated above in a satisfactory manner.

According to the present invention, this object is achieved by means of communication apparatus having the characteristics indicated in the claims which follow the present description.

Further advantages and characteristics of the present invention will become clear from the following detailed description given with the aid of the appended drawings, provided by way of non-limiting example, in which:

Figure 1 is a schematic view of the human skin illustrating the principle of the operation of the apparatus according to the present invention,

Figure 2 is a schematic block diagram showing the principle of the operation of the apparatus according to the invention,

Figure 3 is a schematic block diagram of the communication apparatus according to the invention.

The human skin has a layered structure which consists of two main layers: a cellular outer covering known as the epidermis and a layer of connective tissues known as the dermis. The epidermis is composed of cells and cell debris. The continual reproduction of epithelial cells at the junction between the epidermis and the dermis causes migration of cells towards the outer surface of the skin. The cells are keranitized as they move outwards.

The stages of the transformation process of the cells characterize the five layers into which the epidermis is divided; the germinative layer (which is in contact with the dermis), the spinous layer, the granular layer, the transparent layer, and the cornified layer. The cells die even before they enter the transparent layer.

The dermis in turn is constituted by a dense network of fibrous proteins such as collagen, reticulin and elastin, permeated with micropolysaccharides.

From an electrical point of view, the structure of the human skin is comparable to that of a waveguide. More specifically, given its structure, the skin behaves like a planar waveguide. The cornified layer of the epidermis behaves, owing also to perspiration, as an electrical conductor, the transparent layer, which is constituted exclusively by dead cells, has the behaviour of a dielectric material, and the layers below the epidermis, that is, the dermis and the rest of the body, which are bathed abundantly with blood have the behaviour of an electrical conductor.

In a structure of this type, an electromagnetic wave is guided along the surfaces of the conductors and of the dielectric interposed between them. The electromagnetic wave is guided by means of an intimate connection between the fields of the wave and the currents and the charges at the boundary.

Energy is transmitted by means of the electromagnetic fields of the wave in the region of the dielectric disposed between the lateral boundary surfaces which are the main factor in determining the particular characteristics of the wave.

This phenomenon can be investigated theoretically by determining the solutions to the equations of the waves which satisfy the boundary conditions imposed by the conductive walls and by the dielectric of the waveguide.

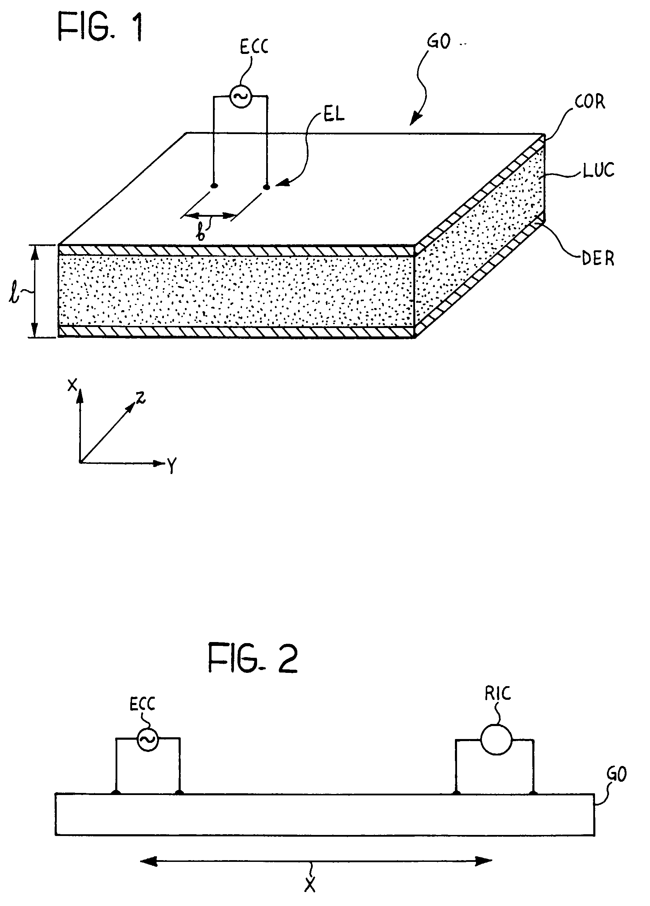

For a better understanding, the theory of the electromagnetic propagation guided in the structure constituted by the human skin, which is substantially comparable to a planar waveguide, will now be analysed briefly. This analysis will be carried out with the aid of Figures 1 and 2.

As can be seen, Figure 1 shows a portion of human skin GO shown schematically as a planar waveguide. Three different layers which make up the planar waveguide have been shown in the human skin GO. As stated above, these layers are: the conductive, cornified, surface layer COR; the intermediate, transparent, dielectric layer LUC; and the lower, conductive dermis layer DER. Naturally, this figure is schematic and not to scale since it serves solely to illustrate the principle of operation.

The distance I between the two conductive layers COR and DER and the orientations of the Cartesian axes X, Y, Z used in the theoretical discussion are also indicated in Figure 1. The drawing also shows an excitation device ECC connected to the surface of the skin GO and, more specifically, to the conductive surface layer COR, by means of two electrodes EL. The distance between the electrodes EL of the excitation device ECC is indicated b.

Figure 2, on the other hand, shows schematically a portion of skin GO to which an excitation device ECC, as in Figure 1, and a receiver device RIC are applied. The receiver device RIC can detect the electrical signals emitted by the excitation device ECC. The excitation device ECC and the receiver device RIC are separated by a distance indicated x.

The analysis can be developed by considering, for the wave, a propagation factor $\gamma$ containing the data relating to the attenuation, and to the phase and group velocities of the wave, and investigating the Maxwell equations in orthogonal coordinates.

The equations of the rotations developed for the dielectric or LUC region, give the following equations:

$$\overline{V}x\vec{E} = -j\omega\mu\vec{H} \qquad (1)$$

$$\frac{\partial E_z}{\partial y} + \gamma E_y = -j\omega\mu H_x \qquad (1.1)$$

$$-\gamma E_x - \frac{\partial E_z}{\partial x} = -j\omega\mu H_y \qquad (1.2)$$

$$\frac{\partial E_y}{\partial x} - \frac{\partial E_x}{\partial y} = -j\omega\mu H_z \qquad (1.3)$$

$$\overline{V}x\vec{H} = j\omega\varepsilon\vec{E} \qquad (2)$$

$$\frac{\partial H_z}{\partial y} + \gamma H_y = j\omega\varepsilon E_x \qquad (2.1)$$

$$-\gamma H_x - \frac{\partial H_z}{\partial x} = j\omega\varepsilon E_y \qquad (2.2)$$

$$\frac{\partial H_y}{\partial x} - \frac{\partial H_x}{\partial y} = j\omega\varepsilon E_z \qquad (2.3)$$

from which, with $K = \omega\sqrt{\mu\varepsilon}$ (wave vector):

$$H_x = \frac{1}{\gamma^2 + k^2}\left(j\omega\varepsilon \frac{\partial E_z}{\partial y} - \gamma \frac{\partial H_z}{\partial x}\right) \quad (3)$$

$$H_y = -\frac{1}{\gamma^2 + k^2}\left(j\omega\varepsilon \frac{\partial E_z}{\partial x} + \gamma \frac{\partial H_z}{\partial y}\right) \quad (4)$$

$$E_x = -\frac{1}{\gamma^2 + k^2}\left(\gamma \frac{\partial E_z}{\partial x} + j\omega\mu \frac{\partial H_z}{\partial y}\right) \quad (5)$$

$$E_y = \frac{1}{\gamma^2 + k^2}\left(-\gamma \frac{\partial E_z}{\partial y} + j\omega\mu \frac{\partial H_z}{\partial x}\right) \quad (6)$$

in which the coefficients $H_x$, $H_y$, $E_x$, $E_y$, are functions solely of the variables x and y, the dependence on time and on the direction z of propagation of the wave being contained in the propagation factor $\gamma$.

The total intensities of the electric and magnetic fields in the region of the dielectric LUC, without charges, between the conductive walls COR and DER have to satisfy the wave equations:

$$\overline{V}^2_{xy}\vec{E} = -(\gamma^2 + K^2)\vec{E} \quad (7)$$

$$\overline{V}^2_{xy}\vec{H} = -(\gamma^2 + K^2)\vec{H} \quad (8)$$

If a voltage generator, that is, the excitation device ECC, is applied to the cornified layer COR of the skin GO, a current flows thereon whilst an electrical polarization phenomenon is present on the underlying conductive surface. An electric field and a magnetic field are thus formed perpendicular to one another and an electromagnetic wave is propagated in a direction perpendicular to those of the fields (a transverse electromagnetic wave, TEM).

This is the conventional situation for electromagnetic propagation in a waveguide with parallel and infinite planes, which is an optimal approximation of a structure formed by two parallel conductive cylinders with extremely close radii (which in turn is a good schematic representation of the skin GO of the human body).

The electric and magnetic fields are expressed in phasor form by the expressions:

$$E_x(z) = E_o e^{\pm jkz} \quad (9)$$

$$\eta H_y(z) = \pm E_o e^{\pm jkz} \quad (10)$$

$$\eta = \sqrt{\frac{\mu}{\varepsilon}} \quad (11)$$

where $\eta$ is the characteristic impedance of the dielectric LUC.

The voltage between the two conductive planes COR and DER is expressed, as a function of z, by the equation:

$$V(z) = -\int_o^l E_x(z)\,dx = -lE_o e^{\pm jkz} \quad (12)$$

l being the thickness of the dielectric LUC.

The current per unit width on the upper cornified layer COR is:

$$J_z = -H_y \quad (13)$$

Considering the distance b in the direction y, the current on the cornified layer COR is:

$$I(z) = -bH_y = \pm\frac{bE_o}{\eta} e^{\pm jkz} \quad (14)$$

whilst the impedance is given by:

$$Z_o = \eta \frac{\theta}{b} \quad (15)$$

The power transferred by the electromagnetic wave can be calculated directly from the fields. In order to do this, it is necessary to calculate the Poynting vector and to integrate it in the dielectric region LUC between the two conductive planes COR and DER. In this case, the Poynting vector has a component solely along the z axis and the mean power transmitted by a single wave travelling in the positive direction is therefore given by:

$$W_T = b\int_o^l \frac{1}{2}Re(E_x H_y^*)\,dx = \frac{bl}{2}\frac{E_o^2}{\eta} \quad (16)$$

For the human skin GO, the dielectric constant at 100 KHz is greater than 2000 whereas, for the magnetic susceptibility, reference may be made to that of water (-2.2 x 10$^{-9}$) giving, for the expression for the power transferred, the following equation:

$$W_T = 0,1187 \cdot \frac{b}{2a} \cdot V_o^2 \quad (17)$$

where b >> 1.

With reference to the expression for the wave vector k, a voltage $v_1$ generated at a given point by the excitation device ECC can be detected as a voltage $v_2$ at another peripheral point, for example, by the receiver device RIC, at a distance x from the first point, as shown in Figure 2, attenuated at a working frequency, for example, of 100 KHz, according to the equation:

$$V_2 = V_1 \cdot \exp[(-1.49 \cdot 10^{-2}) \cdot x] \qquad (18)$$

so that, at a distance, for example, of 2 metres, it is found that: $V_2 = 0.97 \cdot V_1$.

Communication apparatus using the human body as a transmission line has thus been developed with the use of this principle. More specifically, the communication apparatus according to the present invention uses the human skin GO as a planar waveguide which permits the propagation of carried electromagnetic waves and therefore acts as a transmission line. Although this transmission line, constituted by the human skin GO, has far from ideal characteristics, it can however permit effective and reliable communication between the devices.

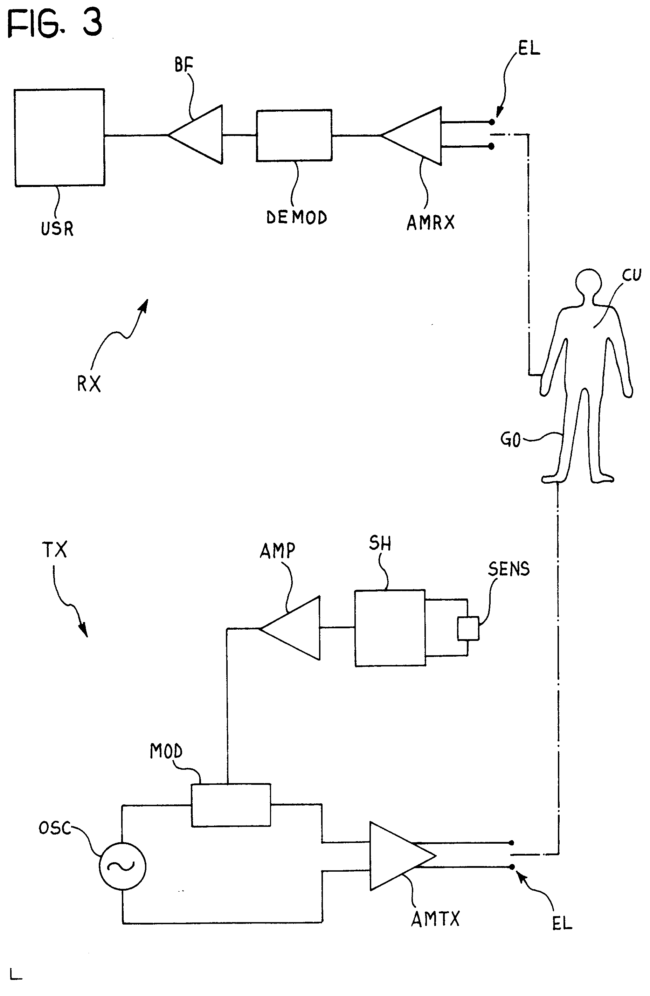

This apparatus will now be described with the aid of Figure 3, which is a schematic block diagram of the apparatus.

As can be seen from Figure 3, the apparatus is constituted essentially by a transmitter device or module TX and a receiver device or module RX. The modules TX and RX communicate with one another with the use of the skin GO of a human body CU as a transmission line. The transmitter module TX comprises an oscillator circuit OSC which can generate a carrier frequency or wave for transmission by the planar waveguide constituted by the skin GO.

Naturally, for effective transmission, this carrier wave generated by the oscillator OSC has to have a frequency compatible with the characteristics of the human skin GO. For example, the carrier wave may have a frequency of the order of 100 KHz. The carrier frequency generated by the oscillator OSC is applied to the skin GO by means of a transmission amplifier AMTX, the output of which is connected to two electrodes EL to be placed in contact with the skin GO. These electrodes EL may be connected either galvanically or capacitively to the upper layer, that is, the cornified layer COR of the skin GO.

In tests carried out by the Applicant, both electrodes of the type used for recording electrocardiograms, that is, electrodes which are placed in galvanic contact with the skin GO, and metal or, in any case, electrically-conductive elements, insulated from the skin GO, that is, connected capacitively to the skin GO, have been used successfully. The metal elements may, for example, be flexible metal plates or meshes facing the skin GO but separated therefrom by one or more insulating layers, for example, such as clothing.

In the case of footwear, for example, the metal plates or meshes may be fitted in the sole of the footwear and can be connected capacitively to the skin GO of the sole of the foot via a surface layer of the sole of the shoe which, typically, is insulating, and possibly a sock worn by the person, which is also insulating.

Naturally, since the function of the transmitter module TX is to transmit not only the carrier frequency but also data, it comprises a modulator MOD interposed between the oscillator circuit OSC and the transmission amplifier AMTX. The modulator MOD modulates the carrier frequency on the basis of the data to be transmitted.

This modulation may be of various types, for example, amplitude modulation or frequency modulation. Given the far from ideal characteristics of the transmission medium, that is, the skin GO, it is considered preferable to use frequency modulation.

The data transmitted by the transmitter module TX may also be of various kinds and may be transmitted in either analog or digital format. This data may be constituted, for example, by electrical signals emitted by a biomedical sensor SENS for detecting a physical parameter. The sensor SENS is connected to a signal sampling and/or processing circuit SH. The output of the signal sampling and/or processing circuit SH is connected to an amplifier AMP. A signal is thus emitted at the output of the amplifier AMP and is sent to the modulator MOD for the modulation of the carrier frequency.

The modulated carrier frequency is then applied to the skin GO, as stated above, by means of the electrodes EL connected to the output of the transmission amplifier AMTX. These electromagnetic signals are then propagated over the entire surface constituted by the skin GO of the human body CU. The receiver module RX can therefore be connected to any point of the human body CU and receive the signals transmitted by the transmitter module TX.

The receiver module RX comprises, for this purpose, a receiving amplifier AMRX having its inputs connected to two electrodes EL for application to the skin GO. At the output of the receiving amplifier AMRX there is therefore, essentially, the modulated carrier frequency transmitted by the transmitter module TX.

The output of the receiving amplifier AMRX is therefore connected to a demodulator DEMOD which isolates the modulating signal, that is, the data signal to be received. This data signal is sent to a low-frequency amplifier BF. The output of the low-frequency amplifier BF in which the data signal is available can thus be sent to a user device USR.

The user device USR may be any device for receiving the data signal. It may therefore be, for example, an electronic processor for processing the data signal transmitted, or a display device for displaying the data transmitted.

Since the communication apparatus according to the invention operates with very low voltages and powers and relatively high frequencies, it is not in any way harmful or annoying to the person whose skin GO is used as a transmission line. Moreover, since the transmission is guided, it is free of the interference problems discussed above with reference to radio communication devices.

Naturally, the principle of the invention remaining the same, the details of construction and forms of embodiment may be varied widely with respect to those described and illustrated, without thereby departing from the scope of the present invention as defined in the attached claims.

**Claims**

1. Transmission apparatus comprising a transmitter module (TX) and a receiver module (RX) communicating with one another by means of a transmission line (GO),
characterized in that the transmission line (GO) is constituted by the skin (GO) of a human body (CU) acting as a planar waveguide,
the transmitter module (TX) and the receiver module (RX) being configured in a manner such that they can be connected to a surface layer (COR) of the skin (GO).

2. Apparatus according to Claim 1, characterized in that it is configured for using, as a transmission medium, in the form of a planar waveguide, a structure formed by three layers (COR, LUC, DER) of the human skin (GO), the three layers (COR, LUC, DER) comprising two layers (COR, DER) essentially having the characteristics of an electrically-conductive material, and one layer (LUC) essentially having the characteristics of a dielectric material, interposed between the two conductive layers (COR, DER).

3. Apparatus according to Claim 2, characterized in that the two conductive layers (COR, DER) of the skin are an upper layer called the cornified layer (COR) and a lower layer called the dermis (DER), and the dielectric layer is an intermediate layer called the transparent layer (LUC).

4. Apparatus according to any one of Claims 1 to 3, characterized in that it operates at a frequency compatible with the characteristics of the planar waveguide constituted by the human skin (GO).

5. Apparatus according to Claim 4, characterized in that it operates at a frequency substantially of the order of 100 KHz.

6. Apparatus according to any one of Claims 1 to 5, characterized in that it uses a frequency-modulated carrier wave.

7. Apparatus according to any one of Claims 1 to 6, characterized in that the transmitter module (TX) and the receiver module (RX) are connected to the conductive surface layer (COR) by means of electrodes (EL).

8. Apparatus according to Claim 7, characterized in that the electrodes (EL) are connected galvanically to the surface layer (COR).

9. Apparatus according to Claim 7, characterized in that the electrodes (EL) are connected to the surface layer (COR) capacitively.

10. Apparatus according to any one of Claims 7 to 9, characterized in that each of the transmitter module (TX) and the receiver module (RX) comprises two electrodes (EL).

11. Apparatus according to any one of Claims 1 to 10, characterized in that at least one of the transmitter module (TX) and the receiver module (RX) is incorporated in footwear.

12. Apparatus according to Claim 11, characterized in that the at least one of the transmitter module (TX) and the receiver module (RX) comprises two electrodes (EL), the two electrodes (EL) being:

    - incorporated in a sole of the footwear,
    - of substantially planar shape, and
    - connectible capacitively to the surface layer (COR) of the skin (GO).

13. Apparatus according to Claim 12, characterized in that the two electrodes (EL) are constituted by metal plates or meshes.

14. Footwear, characterized in that it comprises at least one module (TX), RX) of communication apparatus using the skin (GO) of a human body (CU) as a transmission line, according to any one of Claims 11 to 13.

# FIG. 1

# FIG. 2

# FIG. 3

European Patent
Office

**EUROPEAN SEARCH REPORT**

Application Number

EP 97 11 4064

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X<br>A | GB 2 129 176 A (ROUNDEL ELECTRONICS)<br>* page 1, line 114 - page 3, line 122; tables 1-6 * | 1,5,7<br>2,4,6,<br>8-10 | A61B5/00 |
| X,P<br>A | GB 2 306 725 A (ROBINSON ALAN WILLIAM)<br>* page 1, line 1 - page 12, line 16; tables 1-5 * | 1,7<br>2,4,6,<br>8-10 | |
| X<br><br><br><br>A | PATENT ABSTRACTS OF JAPAN<br>vol. 010, no. 205 (E-420), 17 July 1986<br>& JP 61 046639 A (MATSUSHITA ELECTRIC WORKS LTD), 6 March 1986,<br>* abstract * | 1,7<br><br><br><br>4,8 | |

TECHNICAL FIELDS
SEARCHED        (Int.Cl.6)

A61B

The present search report has been drawn up for all claims

| Place of search | Date of completion of the search | Examiner |
|---|---|---|
| BERLIN | 3 November 1997 | Weihs, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)